# EUROPEAN PATENT SPECIFICATION

(11) **EP 1 467 821 B1**
(45) Date of publication and mention of the grant of the patent: **11.03.2009**
(21) Application number: 03701598.9
(22) Date of filing: 23.01.2003
(51) Int. Cl.: B05B 11/02, B01F 13/00, B01F 11/00, B01F 13/06

(54) **DISPENSING DEVICE**
SPENDEVORRICHTUNG
DISPOSITIF DE DISTRIBUTION

(30) Priority: 23.01.2002 GB 0201507
(43) Date of publication of application: 20.10.2004
(73) Proprietor: Consort Medical PLC, Breakspear Way Hemel Hempstead Hertfordshire HP2 4UL (GB)
(72) Inventor: WRIGHT, Andrew, David, Norfolk PE31 7EH (GB)
(74) Representative: Thomson, Neil David
(86) International application number: PCT/GB2003/000287
(87) International publication number: WO 2003/061844

(56) References cited:
- GB-A- 1 550 016
- US-A- 5 193 907

## Description

The invention relates to dispensing devices and, particularly to dispensing devices for dispensing medicaments in suspension in a spray form.

Delivery systems are known for administering liquids in spray form. One such system is described in published application WO91/15303. A problem has been found with dispensing liquids, including medicaments, which are formed from suspensions using such devices. It is a requirement of such devices that the liquid medicament is stored without any air being present in the medicament cartridge or medicament vial. This is due to the fact that the air can cause degradation of the medicament, because of the dangers of injecting air into the patient, especially where the medicament is used intravenously, and also the inconsistency in shot weight which occurs when air is present. As a result, the vials or cartridges or other containers for the medicament are formed with no air space. A problem has been found with dispensing suspensions from such vials or cartridges since it has been found that over time, the suspended element of the suspension settles out from the liquid carrier. In order for a correct dosage to be dispensed, it is therefore necessary to agitate the medicament to resuspend the medicament in the liquid carrier. This is not easy to achieve efficiently with many suspensions in a medicament vial or cartridge where there is no air space or other head space.

The present invention provides a method of dispensing a liquid suspension from a reservoir of a liquid suspension held in a delivery system, wherein the reservoir of liquid suspension is of the type which is normally isolated from atmosphere, comprising the steps of:
a) increasing the volume of the reservoir above an initial volume so as to reduce the pressure in the reservoir to below atmospheric;
b) agitating the liquid suspension;
c) reducing the volume of the reservoir to the initial volume; and
d) subsequently dispensing at least a portion of the liquid suspension from the reservoir.

The present invention also provides a delivery system for dispensing a liquid suspension comprising a reservoir of the liquid suspension of the type which is normally isolated from atmosphere, means for creating at least a partial vacuum in the reservoir of liquid suspension and means for dispensing at least a portion of the liquid from the reservoir subsequent to agitation of the liquid suspension.

The present invention also provides use of the delivery system.

An embodiment of the present invention will now be described, by way of example only, with reference to the accompanying drawing, which is a sectional elevation through a dispensing device according to the invention.

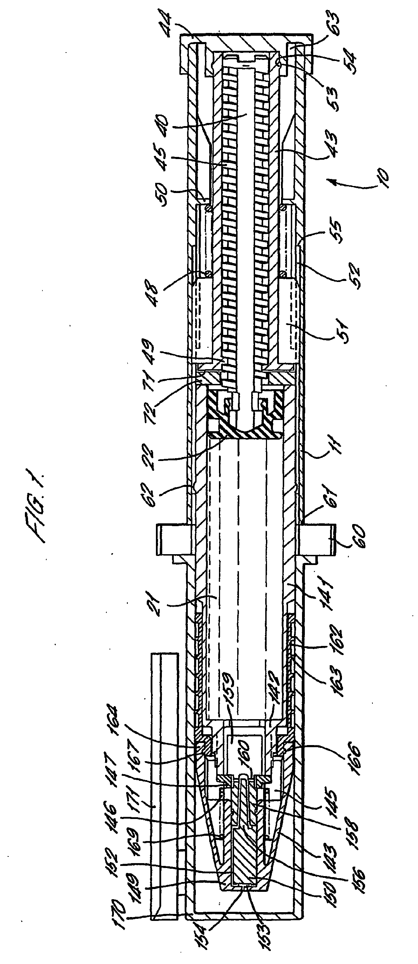

Referring to Figure 1, there is illustrated a dispensing device intended for dispensing a liquid medicament in the form of a spray. The device forms a delivery system which is particularly suitable for nasal administration of a medicament.

The dispensing device comprises a drive unit 10, a dispenser housing 11, a nozzle 143 and a protective cap 170.

The dispenser housing 11 is a cylindrical tube. A generally cylindrical housing 141 is received slidingly inside, and connected to, the dispenser housing 11 by means of a snap-fit co-operating feature 62. The generally cylindrical housing 141 has a stepped end 142 which is generally closed off and connects to the nozzle 143 of the device and includes at its other end a piston 22 which is sealingly received in the cartridge 141 so as to isolate the contents of the cartridge 141 from atmosphere when the nozzle 143 is in the closed position. The drive unit 10 is arranged to move the piston 22 to the left as viewed in Figure 1 by pre-selected distances corresponding to pre-selected doses of medicament stored in the device which are to be dispensed. The operation of the drive unit 10 will be described further below.

The stepped end 142 of the generally cylindrical housing 141, which serves as a medicament cartridge, is closed off by a valve cap 145 having a central aperture 146 fixed to the end of the cartridge 141 and trapping a valve seat 147 between the valve cap 145 and the open end of stepped portion 142 of the cartridge 141.

The nozzle 143 of the device comprises outer and inner nested components 149 and 150 which define between them a flow path for medicament to be dispensed. The nozzle 143 is of the type designed to dispense a pressurised liquid passing through the nozzle in the form of a spray. The construction of the nozzle 143 may be, for example, as described in published European patent application 0 308 100. In summary, an axially extending channel 152 is defined between the inner and outer components 149, 150 leading to a swirl chamber 153 and outlet orifice 154 of the device. The inner end of the inner nested component 150 includes a tapered peg 156 which is fitted in a tapered bore of a valve core 158. The valve core 158 includes an enlarged portion 159, the shoulder of which engages the valve seat 147 to isolate the contents of cartridge 141 from the atmosphere when the device is not in use.

When the portion 159 is unseated from valve seat 147, medicament may flow into the nozzle 143 through side holes 160 in the valve core 158 and thence through a flow path formed between tapered peg 156 and the bore in which it seats. This connects in turn with axial bore 152 in nozzle 143.

The inner and outer nested components 149, 150 of the nozzle 143 and the valve core 158 are fixed together to move as a single component. The assembly of nozzle and valve core is moved by a slider 162 including finger grips 163 which is fixed to a skirt portion 164 of the outer component 149 of the nozzle assembly. As shown in Figure 1, the attachment of the slider 162 to the skirt portion 164 is by means of an inter-engaging annular bead and groove 166, 167 which snap-fit together. The assembly of nozzle 143 and valve stem 158 is urged into its normally closed position by spring 169 located between an inner surface of outer nozzle component 149 and valve cap 145.

The dispensing device 140 also includes a cap 170 which fits over the cartridge 141 enclosing the nozzle assembly 143 and includes a clip 171 similar to a pen cap.

In use of the device 140, the cap 170 is first removed. The device is then pre-loaded (as will be described below) to dispense a pre-selected quantity of medicament by selecting the required dose on the drive unit 10. The user then applies nozzle 143 to the point at which the medicament is to be introduced (for example, a nostril) and slides the slider 162 relative to the cartridge 141 thereby unseating the valve stem 158 from the valve seat 147 and allowing a portion of the medicament stored in the reservoir of the cartridge 141 to be dispensed.

The drive unit 10 of the dispensing device is located in housing 11. The end of housing 11 remote from nozzle 143 is closed off a cap 44. The housing 11 is open at the other end 61 for receiving the cartridge 141 as described above. A plunger 40 extends from end cap 44 towards piston member 22 and the drive unit 10 is operable to cause the plunger 40 to move axially towards nozzle 143 by a fixed distance determined by a dose selector (not shown) which is incorporated in end cap 44. The end cap 44 is rotatably mounted on the end of the housing 11 remote from plunger 40. Axial movement of the plunger 40 by said fixed distance causes the piston 22 to be urged to move the same distance to dispense the selected dose of the liquid medicament as described above.

The plunger 40 has an integrally formed thread 45 of a large lead angle. The plunger 40 slides axially within a plunger guide 43 which comprises inwardly directed teeth 49 which engage the thread 45 of the plunger 40. The plunger guide 43 is coupled to end cap 44 by co-operating bead and groove formations 54 and 53 which,snap-fit together. The plunger guide 43 is rotatably fixed relative to the end cap 44 such that rotation of end cap 44 causes plunger guide 43 also to rotate. Rotation of plunger guide 43 in turn urges plunger 40 to move axially towards nozzle 143 by means of the engagement between teeth 49 and thread 45.

The plunger guide 43 comprises a number of circumferentially spaced ribs 51 which extend from an outer surface of the plunger guide 43 towards the inner wall of housing 11. The inner wall of housing 11 is provided with a matching number of T-shaped channels 52 in which the distal edges of ribs 51 are received. As shown in Figure 1, the channels 52 extend axially from open end 61 to a point axially to the right of ribs 51 when in the position shown in Figure 1.

A spring 48 is provided seated between an end face of the ribs 51 nearest of the end cap 44 and a spring seat 50 provided on an inwardly directed flange of the housing 11. The spring 48 acts to urge the plunger guide 43 towards the left as shown in Figure 1.

The plunger guide 43 further comprises an end face 71 directed towards nozzle 143 which is provided with a series of teeth (not shown) which engage matching teeth on a ratchet disc 72 located between the plunger guide 43 and the cartridge 141. The ratchet disc 72 is rotationally fixed relative to cartridge 141. The matching sets of teeth are directed relative to one another such that relative rotation between the plunger guide 43 and the ratchet disc 72 is possible in only one direction.

In use to dispense a dose the end cap 44 is rotated which in turn rotates plunger guide 43 and also housing 11 by means of the engagement of the ribs 51 in the channels 52. The ratchet disc 72 and teeth on the end face 71 of the plunger guide 43 prevent rotation of the plunger guide 43 in the opposite direction. Rotation of the plunger guide 43 acts on the thread 45 of the plunger 40 through the teeth 49. Due to the incompressibility of the liquid medicament 21, the piston member 22 is unable to move towards the left as shown in Figure 1 whilst the nozzle 143 is in the closed position. Hence, rotation of end cap 44 causes the plunger guide 43 and end cap 44 to move to the right as shown in Figure 1 so as to accommodate the relative rotational movement between the thread 45 and teeth 49. As a result, the end cap 44, plunger guide 43 and ribs 51 move towards the right as shown in Figure 1, compressing the spring 48 between the end face of ribs 51 and the spring seat 50 so as to charge the drive unit 10 with stored spring energy. Co-operating formation 62 prevents housing 11 moving to the left as viewed in Figure 1 under action of spring 48 as the spring force of spring 48 is insufficient to unseat the co-operating formation 62. An end face 55 of the T-shaped channels 52 acts as a limiter on the axial movement of the ribs 51 towards the right as shown in Figure 1 so as to limit the energy stored in spring 48. Alternatively, the dose selector incorporated in end cap 44 may comprise means for limiting the rotation of end cap 44 during charging.

Once charged, the nozzle 143 is operated as described above by movement of the slider 162. At the point when the flow path to the outlet orifice 145 opens, the liquid medicament 21 is able to be dispensed, accommodating movement of the piston member to the left as shown in Figure 1. At this point, spring 48 urges the ribs 51, plunger guide 43, dose selector 44 and plunger 40 to move to the left with piston member 22 to dispense the pre-selected dose of liquid medicament 21. The device is then ready to be re-charged for a subsequent actuation.

A problem has been found with dispensing liquid medicaments which are formed from suspensions using devices such as the one described above. It is a requirement of such devices that the liquid medicament 21 is stored without any air being present in the medicament cartridge 141 or medicament vial. This is due to the fact that the air can cause degradation of the medicament, because of the dangers of injecting air into the patient, especially where the medicament 21 is used intravenously, and also the inconsistency in shot weight which occurs when air is present. As a result, the vials or cartridges or other containers for the medicament 21 are formed with no air space. A problem has been found with dispensing suspensions from such vials or cartridges since it has been found that over time, the suspended element of the suspension settles out from the liquid carrier. In order for a correct dosage to be dispensed, it is therefore necessary to agitate the medicament to resuspend the medicament in the liquid carrier. This is not easy to achieve efficiently with many suspensions in a medicament vial or cartridge where there is no air space or other head space, especially where the user is elderly or infirm.

The present invention overcomes this problem by providing means for creating a vacuum or partial vacuum in the normally air free medicament vial or cartridge.

Referring to Figure 1, a portion of the cartridge 141 is provided with an external screw thread on which is mounted a threaded nut 60 which abuts the open end 61 of the housing 11. In addition, end cap 44 is provided with a circumferential channel 63 in which is received the opposite end of the housing 11. In use, before operating the end cap 44 to charge the device for dispensation, the threaded nut 60 is rotated so as to move the threaded nut 60 to the right as viewed in Figure 1 along the external thread of the cartridge 141. The movement of the threaded nut 60 to the right as shown in Figure 1 forces the housing 11 and, by means of the engagement of the end of housing 11 in the circumferential channel 63, the end cap 44 to the right as viewed in Figure 1. The rotation of the nut 60 provides sufficient force to unseat the co-operating formation 62 to allow the housing 11 to slide axially relative to the cartridge 141. The movement of the housing 11 in turn causes the piston member 22 to be moved to the right as viewed in Figure 1 due to the engagement of the plunger 40, piston guide 43 and end cap 44. Consequently, operation of the threaded nut 60 produces an increase in volume of the cartridge and at the same time a reduction in pressure in the cartridge 141 below atmospheric since, with the nozzle 143 closed, the cartridge 141 is isolated from atmosphere. As such, a partial vacuum is created in the cartridge 141. The user then agitates the cartridge 141 by shaking the device to resuspend the suspension. The threaded nut 60 is then rotated in the opposite sense to return it to its original position. At the same time the suction effect of the partial vacuum in the cartridge 141 draws the piston member 22 back to its original position causing the co-operating formation 62 to re-engage. To aid re-engagement the faces of the co-operating formation contacting one another on re-engagement may be tapered so as to urge the formations to ride over one another.

Once the co-operating formations 62 are reengaged, the device is ready to be charged and fired in the same manner as described above.

The present invention is not limited to the device described above which is provided as merely an illustrative example. The present invention provides equal application with both single dose and multiple dose devices. The medicament may be provided in a replaceable cartridge, vial or other container. Alternatively, the medicament may be provided in a chamber formed as part of the housing of the device, especially where the device is a single dose dispenser.

Other drive units may be used to power the dispensation of the medicament. One example is described in published European Patent application 0338806.

Other means of sealing the nozzle 143 and other nozzle types may be used without departing from the scope of the present invention as defined in the claims.

The delivery system may be adapted for other types of administration, for example oral or sublingual.

## Claims

1. A method of dispensing a liquid suspension from a reservoir of a liquid suspension held in a delivery system, wherein the reservoir of liquid suspension is of the type which is normally isolated from atmosphere, comprising the steps of:
a) increasing the volume of the reservoir above an initial volume so as to reduce the pressure in the reservoir to below atmospheric;
b) agitating the liquid suspension;
c) reducing the volume of the reservoir to the initial volume; and
d) subsequently dispensing at least a portion of the liquid suspension from the reservoir.

2. The method of claim 1 wherein at least a partial vacuum is created in the reservoir during step a).

3. The method of claim 2 wherein the delivery system comprises a piston member engageable in the reservoir and the at least partial vacuum is created by partially withdrawing the piston member from the reservoir.

4. The method of any of claims 1 to 3 wherein the liquid is dispensed by pressurising the reservoir to a level above atmospheric in step d).

5. A delivery system for dispensing a liquid suspension comprising a reservoir of the liquid suspension of the type which is normally isolated from atmosphere, means for creating at least a partial vacuum in the reservoir of liquid suspension and means for dispensing at least a portion of the liquid from the reservoir subsequent to agitation of the liquid suspension.

6. The delivery system of claim 5 wherein the means for creating at least a partial vacuum in the reservoir of liquid suspension comprises means for increasing the volume of the reservoir while maintaining the reservoir isolated from atmosphere.

7. The delivery system of claim 6 further comprising a piston member engageable in the reservoir.

8. The delivery system of claim 7 further comprising means for at least partially withdrawing the piston member from the reservoir so as to increase the volume of the reservoir.

9. The delivery system of claim 8 wherein the means for withdrawing the piston member comprises a rotatable member rotation of which urges the piston member to withdraw from the reservoir.

10. The delivery system of any of claims 5 to 9 wherein the reservoir contains a single dose of the liquid suspension.

11. The delivery system of claim 10 wherein the reservoir is integrally formed with the delivery system.

12. The delivery system of any of claims 5 to 9 wherein the reservoir contains multiple doses of the liquid suspension.

13. The delivery system of claim 12 wherein the reservoir is integrally formed with the delivery system.

14. The delivery system of claim 12 wherein the reservoir is a replaceable component of the delivery system.

15. The delivery system of claim 14 wherein the reservoir is a vial, ampule or similar cartridge.

16. The delivery system of any of claims 5 to 15 wherein the delivery system is adapted for nasal use.

17. The delivery system of any of claims 5 to 15 wherein the delivery system is adapted for oral use.

18. Use of the delivery system of any of claims 5 to 17.

## Patentansprüche

1. Verfahren zum Abgeben einer Flüssigkeitssuspension aus einem Flüssigkeitssuspensionbehälter, der in einem Zuführsystem getragen ist, wobei der Flüssigkeitssuspensionbehälter von der Art ist, die normalweise von der Atmosphäre isoliert ist, umfassend die Schritte von:
a) Erhöhen des Volumens des Behälters über einen Initialvolumen, so dass der Druck im Behälter unter den Atmosphärendruck reduziert wird;
b) Vermischen der Flüssigkeitssuspension;
c) Reduzieren des Volumens des Behälters auf das Initialvolumen; und
d) anschließendes Abgeben wenigstens eines Teils der Flüssigkeitssuspension aus dem Behälter.

2. Verfahren nach Anspruch 1,
wobei wenigstens ein teilweises Vakuum im Behälter während Schritt a) erzeugt wird.

3. Verfahren nach Anspruch 2,
wobei das Zuführsystem ein Kolbenelement umfasst, das in den Behälter eingreifen kann, und wobei das wenigstens teilweise Vakuum durch teilweises Zurückziehen des Kolbenelements aus dem Behälter erzeugt wird.

4. Verfahren nach einem der Ansprüche 1 bis 3,
wobei die Flüssigkeit durch unter Druck setzen des Behälters auf ein Niveau über den Atmosphärendruck in Schritt d) abgegeben wird.

5. Zuführsystem zum Abgeben einer Flüssigkeitssuspension umfassend:
einen Flüssigkeitssuspensionbehälter von der Art, die normalweise von der Atmosphäre isoliert ist, ein Mittel zum Erzeugen von einem wenigstens teilweisen Vakuum im Flüssigkeitssuspensionbehälter und ein Mittel zum Abgeben von wenigstens einem Teil der Flüssigkeit aus dem Behälter anschließend an ein Vermischen der Flüssigkeitssuspension.

6. Zuführsystem nach Anspruch 5,
wobei das Mittel zum Erzeugen des wenigstens teilweisen Vakuums im Flüssigkeitssuspensionbehälter ein Mittel zum Erhöhen des Volumens des Behälters umfasst, während der Behälter von der Atmosphäre isoliert gehalten wird.

7. Zuführsystem nach Anspruch 6,
das weiter ein Kolbenelement umfasst, das im Behälter eingreifen kann.

8. Zuführsystem nach Anspruch 7,
das weiter Mittel zum wenigstens teilweisen Zurückziehen des Kolbenelements aus dem Behälter umfasst, um so das Volumen im Behälter zu erhöhen.

9. Zuführsystem nach Anspruch 8,
wobei das Mittel zum Zurückziehen des Kolbenelements ein drehbares Element umfasst, dessen Drehung das Kolbenelement dazu treibt, sich aus dem Behälter zurückzuziehen.

10. Zuführsystem nach einem der Ansprüche 5 bis 9,
wobei der Behälter eine einzelne Dosis der Flüssigkeitssuspension enthält

11. Zuführsystem nach Anspruch 10,
wobei der Behälter integral mit dem Zuführsystem gebildet ist.

12. Zuführsystem nach einem der Ansprüche 5 bis 9,
wobei der Behälter mehrere Dosen der Flüssigkeitssuspension enthält.

13. Zuführsystem nach Anspruch 12,
wobei der Behälter integral mit dem Zuführsystem gebildet ist.

14. Zuführsystem nach Anspruch 12,
wobei der Behälter eine ersetzbare Komponente des Zuführsystem ist.

15. Zuführsystem nach Anspruch 14,
wobei der Behälter ein Fläschchen, eine Ampulle oder ein ähnlicher Einsatz ist.

16. Zuführsystem nach einem der Ansprüche 5 bis 15,
wobei das Zuführsystem zur nasalen Verwendung geeignet ist.

17. Zuführsystem nach einem der Ansprüche 5 bis 15,
wobei das Zuführsystem zur oralen Verwendung geeignet ist.

18. Verwendung des Zuführsystems nach einem der Ansprüche 5 bis 17.

## Revendications

1. Procédé de distribution d'une suspension liquide depuis un réservoir d'une suspension liquide monté dans un système de distribution, dans lequel le réservoir de suspension liquide est du type qui est normalement isolé de l'atmosphère, comprenant les étapes qui consistent :
a) à augmenter le volume du réservoir au-dessus d'un volume initial afin d'abaisser en-dessous de la pression atmosphérique la pression régnant dans le réservoir ;
b) à agiter la suspension liquide ;
c) à réduire le volume du réservoir au volume initial ; et
d) à distribuer ensuite au moins une partie de la suspension liquide depuis le réservoir.

2. Procédé selon la revendication 1, dans lequel au moins un vide partiel est créé dans le réservoir pendant l'étape a).

3. Procédé selon la revendication 2, dans lequel le système de distribution comporte un élément à piston pouvant être engagé dans le réservoir et le vide au moins partiel est créé en retirant partiellement l'élément à piston du réservoir.

4. Procédé selon l'une quelconque des revendications 1 à 3, dans lequel le liquide est distribué en mettant sous pression le réservoir à un niveau au-dessus de la pression atmosphérique dans l'étape d).

5. Système de distribution destiné à distribuer une suspension liquide, comportant un réservoir de la suspension liquide du type qui est normalement isolé de l'atmosphère, un moyen pour créer au moins un vide partiel dans le réservoir de la suspension liquide et un moyen pour distribuer au moins une portion du liquide depuis le réservoir après une agitation de la suspension liquide.

6. Système de distribution selon la revendication 5, dans lequel le moyen pour créer au moins un vide partiel dans le réservoir de la suspension liquide comporte un moyen destiné à augmenter le volume du réservoir tout en maintenant le réservoir isolé de l'atmosphère.

7. Système de distribution selon la revendication 6, comportant en outre un élément à piston pouvant être engagé dans le réservoir.

8. Système de distribution selon la revendication 7, comportant en outre un moyen destiné à retirer au moins partiellement l'élément à piston du réservoir afin d'augmenter le volume du réservoir.

9. Système de distribution selon la revendication 8, dans lequel le moyen destiné à retirer l'élément à piston comporte un élément rotatif dont une rotation sollicite l'élément à piston pour le retirer du réservoir.

10. Système de distribution selon l'une quelconque des revendications 5 à 9, dans lequel le réservoir contient une dose unique de la suspension liquide.

11. Système de distribution selon la revendication 10, dans lequel le réservoir est formé d'un seul bloc avec le système de distribution.

12. Système de distribution selon l'une quelconque des revendications 5 à 9, dans lequel le réservoir contient des doses multiples de la suspension liquide.

13. Système de distribution selon la revendication 12, dans lequel le réservoir est formé d'un seul bloc avec le système de distribution.

14. Système de distribution selon la revendication 12, dans lequel le réservoir est une pièce pouvant être remplacée du système de distribution.

15. Système de distribution selon la revendication 14, dans lequel le réservoir est un flacon, une ampoule ou une cartouche similaire.

16. Système de distribution selon l'une quelconque des revendications 5 à 15, lequel système de distribution est conçu pour une utilisation nasale.

17. Système de distribution selon l'une quelconque des revendications 5 à 15, lequel système de distribution est conçu pour une utilisation orale.

18. Utilisation du système de distribution selon l'une quelconque des revendications 5 à 17.
